# EUROPEAN PATENT SPECIFICATION

(11) **EP 3 561 471 B1**
(45) Date of publication and mention of the grant of the patent: **01.12.2021**
(21) Application number: 17882485.0
(22) Date of filing: 31.10.2017
(51) Int. Cl.: G01L 13/00, B01J 4/00, B01J 8/24, C07C 253/24

(54) **PRESSURE DROP CONTROL SYSTEM AND CONTROL METHOD OF FEED DISTRIBUTOR OF A FLUIDIZED BED REACTOR**
DRUCKABFALLSTEUERUNGSSYSTEM UND STEUERUNGSVERFAHREN EINES EINSPEISUNGSVERTEILERS EINES WIRBELBETTREAKTORS
SYSTÈME DE COMMANDE DE CHUTE DE PRESSION ET PROCÉDÉ DE COMMANDE DE DISTRIBUTEUR D'ALIMENTATION D'UN RÉACTEUR À LIT FLUIDISÉ

(30) Priority: 23.12.2016 CN 201611207919
(43) Date of publication of application: 30.10.2019
(73) Proprietor: China Petroleum & Chemical Corporation, Beijing 100728 (CN); Shanghai Research Institute of Petrochemical Technology SINOPEC, Pudong, Shanghai 201208 (CN)
(72) Inventor: ZHAO, Le, Pudong, Shanghai 201208 (CN); WU, Lianghua, Pudong, Shanghai 201208 (CN)
(74) Representative: Rössler, Matthias
(86) International application number: PCT/CN2017/108460
(87) International publication number: WO 2018/113417

(56) References cited:
- WO-A1-2011/090131
- WO-A1-2016/144662
- CN-A- 103 908 930
- CN-U- 204 619 935
- CN-U- 205 797 156
- CN-U- 206 648 775
- US-A- 4 865 540
- US-A- 5 801 265
- US-A1- 2010 080 743
- YU , FEI et al.: "Research of Capacity Expansion for Acrylonitrile Facility of Fushun Petrochemical Company", Contemprary Chemical Industry, vol. 34, no. 5, 31 October 2005 (2005-10-31), pages 345-348,353, XP009515358,

## Description

### Cross-reference to Related Applications

This application claims the priority of Chinese patent application CN201611207919.3, entitled "Pressure drop control system and method for feed distributor of fluidized bed reactor" and filed on December 23, 2016.

### Field of the Invention

The present disclosure relates to a pressure drop control system and a pressure drop control method for a feed distributor of a fluidized bed reactor, and in particular, to a pressure drop control system and a pressure drop control method for a feed distributor of an ammoxidation reactor.

### Background of the Invention

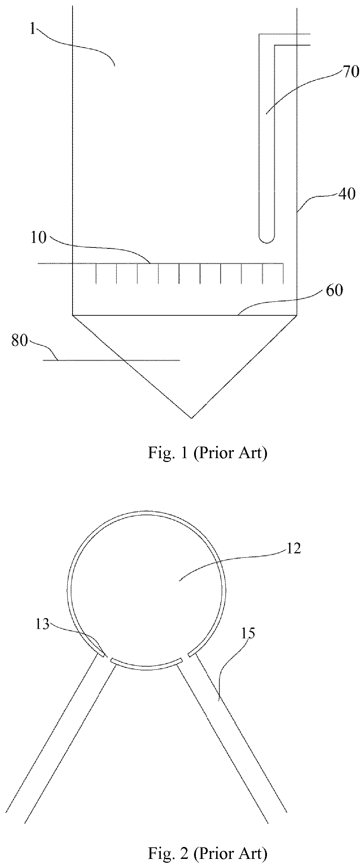

Acrylonitrile is an important chemical material in petrochemical industry. In various countries around the world, acrylonitrile is usually produced by one-step ammoxidation. Specifically, air, propylene, and ammonia mixed in a certain proportion are caused to undergo a selective oxidation reaction between propylene and ammonia in the presence of a catalyst under certain reaction conditions to form acrylonitrile as well as acetonitrile and hydrogen cyanide as byproducts. The reaction can release a large amount of heat. Fig. 1 schematically shows an industrial or (commercial) fluidized bed reactor 1 for producing acrylonitrile. The fluidized bed reactor 1 comprises a reactor wall 40, an air feed port 80, an air distribution plate 60, a propylene and ammonia feed distributor 10, a cooling coil 70, and a cyclone (not shown).

A typical propylene and ammonia feed distributor of a fluidized bed reactor for producing acrylonitrile is usually a multi-tubular distributor which includes an inlet, a main tube, branch tubes, and nozzles. The tubes are in fluid connection. A feed gas enters the distributor through the inlet of the distributor, and flows, from the main tube, to the branch tubes connected with the main tube, and then flows, as shown in Fig. 2, through orifices 13 of the branch 12 to the nozzles 15 and finally to a bed of the fluidized bed reactor. In this way, the feed gas is distributed into the bed by means of the tubes of the distributor.

In propylene ammoxidation conducted in the fluidized bed reactor for producing acrylonitrile, a pressure drop ΔP_{d} of the propylene and ammonia feed distributor of the fluidized bed is a significant parameter. Said pressure drop is, as shown in Fig. 2, a pressure difference between the branch 12 and an end of the nozzle 15. A good design of the pressure drop of the propylene and ammonia feed distributor can enable the reactor to have equal amounts of propylene and ammonia feed gas in unit sectional areas thereof. This requires that all feed gases flowing out of the nozzles of the distributor are equal in amount. Flowing of the feed gas through the orifice causes a local pressure loss which is, as shown in Fig. 2, a pressure difference between the branch 12 and the end of the nozzle 15 (namely a gas outlet of the nozzle 15). This pressure difference is the pressure drop ΔP_{d} of the propylene and ammonia feed distributor.

Properties of the propylene and ammonia feed distributor can directly affect results of the selective oxidation reaction between propylene and ammonia. For uniform distribution of the gas and avoidance of severe gas distribution nonuniformity resulted from certain fluctuation or decrease of load, it is usually required that the distributor have sufficient pressure drop. Scholars tend to take the ratio of the distributor pressure drop ΔP_{d} to the bed pressure drop ΔP_{b} as an object of their researches. The larger the ΔP_{d}/ΔP_{b} ratio is, the more uniform the gas is distributed, but too much pressure drop can lead to large power consumption.

Non-patent literature *A Study on Production Capacity Enhancement of Acrylonitrile Device* (Yu Fei et al, Contemporary Chemical Industry, 2005, volume 34, issue 5, pp. 345-353) discloses that after the production capacity of the acrylonitrile device is enhanced, the flow rate of the gas passing through the orifice is increased which accelerates abrasion of the catalyst, thereby destroying the overall fluidized state. The literature, however, neither discloses a method for measuring the flow rate of the gas passing through the orifice nor a method for measuring the pressure drop of the distributor. In addition, the flow rate of the gas passing through the orifice and the pressure drop of the distributor disclosed by this literature are results of modulation of an ideal state not results of real measurement.

Further, with the continuous improvement to acrylonitrile related technologies, activity index (yield of a target product from conversion of propylene) of catalysts, abrasion resistance of catalyst particles, and separating efficiency of cyclones are all improved. Meanwhile, it is required that a reactor have a higher operating speed so that the production capacity of an acrylonitrile device can be advanced. A low pressure drop of a distributor can easily result in nonuniform distribution of a feed gas or possible backflow of a catalyst caused by low flow rate of the gas passing through an orifice and relatively large orifice diameter, particularly in a large-scale fluidized bed reactor for producing acrylonitrile with a diameter larger than 8.5 m. A low pressure drop of the feed distributor can directly affect reaction results and is not helpful for long-term steady production.

For the purpose of overcoming the above technical problem, it is necessary to provide a pressure drop monitoring system for a feed distributor of a fluidized bed reactor.

WO 2011/090131 A1 discloses a method for supplying a raw material gas to a fluidized reactor through a raw material gas dispersion unit provided in the fluidized reactor and causing a gas-phase reaction of the raw material gas, wherein an inert gas is supplied to the dispersion unit when the pressure loss of the dispersion unit becomes less than 1.0 time of the pressure loss of a fluidized layer.

WO2016/144662 A1 discloses an ammoxidation reactor that includes an outer ring of sets of multistage cyclones suspended in the reactor. Each multistage set of cyclones includes a first stage cyclone having a first stage inlet configured to receive a reactor stream flowing up from a fluidized catalyst bed in the reactor and separate at least a portion of catalyst from the reactor stream. A ratio of square meter of first stage inlet area per square meter of the available cross-sectional area of the reactor is about 0.03 to about 0.05. An ammoxidation process includes reacting a hydrocarbon stream in a fluidized catalyst bed in a reactor to produce a reactor stream. The process further includes separating the catalyst from the reactor stream in an outer ring of sets of multistage cyclones, each multistage set of cyclones including a first stage cyclone having a first stage inlet configured to receive a reactor stream flowing up from a fluidized catalyst bed in the reactor and separate at least a portion of catalyst from the reactor stream. A ratio of cyclone inlet velocity in meters/second to a reactor effluent velocity in meters/second is 15 or greater.

### Summary of the Invention

Directed against the above technical problem, the present disclosure provides a pressure drop control system for a feed distributor of a fluidized bed reactor and a pressure drop control method for a feed distributor of an ammoxidation reactor. The pressure drop control system and method used for measurement of a pressure drop is capable of achieving real-time monitoring of working state of a feed distributor and predetermining the working state of the feed distributor and performing corresponding actions, so that the pressure drop of the feed distributor can be enabled to be in a predetermined scope, whereby the feed distributor is ensured to work normally.

In order to achieve the above technical effects, the present disclosure provides a pressure drop control system for a feed distributor of a fluidized bed reactor. The pressure drop control system comprises: a measuring unit which is configured to measure a pressure drop between a first pressure measurement port and a second pressure measurement port of the feed distributor, the first pressure measurement port being located in an inlet tube of the feed distributor near a reactor wall of the fluidized bed reactor, and the second pressure measurement port being located in an area of the reactor wall between an air distribution plate of the fluidized bed reactor and a gas outlet of a nozzle of the feed distributor; a transfer unit which is configured to carry on signal communications with the measuring unit and collect a signal about the pressure drop measured by the measuring unit; and a processing unit which is configured to monitor whether the feed distributor is working normally or not. When a pressure drop of the feed distributor is in a predetermined scope within which the pressure drop of the feed distributor is 25%-160% of a pressure drop of a bed of the fluidized bed reactor, the processing unit determines that the feed distributor is working normally. When the processing unit determines that the pressure drop of the feed distributor is larger than an upper limit value or is smaller than a lower limit value of the predetermined scope, the processing unit performs corresponding actions so as to restore the pressure drop of the feed distributor to a value within the predetermined scope. The pressure drop control system further comprises a nitrogen purging device. When the processing unit determines that the pressure drop of the feed distributor is smaller than the lower limit value of the predetermined scope, the processing unit turns on the nitrogen purging device so that a nitrogen gas can be fed into the feed distributor, by means of which the pressure drop of the feed distributor can be restored to a value within the predetermined scope.

The pressure drop control system further comprises purging units which are provided at the first pressure measurement port and the second pressure measurement port, respectively.

According to the pressure drop control system, each of the purging units is configured to supply a purging gas having a volumetric flow rate smaller than or equal to 10 Nm³/h.

According to the pressure drop control system, when the pressure drop of the feed distributor is in a predetermined scope within which the pressure drop of the feed distributor is 35%-140% of the pressure drop of the bed of the fluidized bed reactor, the processing unit determines that the feed distributor is working normally.

The pressure drop control system further comprises a nitrogen purging device. When the processing unit determines that the pressure drop of the feed distributor is larger than the upper limit value of the predetermined scope, the processing unit turns on the nitrogen purging device so that the feed distributor can be purged.

According to the pressure drop control system, the measuring unit comprises a first pressure measurement device that is provided at the first pressure measurement port and a second pressure measurement device that is provided at the second pressure measurement port. The transfer unit is configured to carry on signal communications with the first pressure measurement device and the second pressure measurement device respectively, and the processing unit is configured to perform logical differential pressure calculation so as to obtain the pressure drop of the feed distributor.

According to the pressure drop control system, the measuring unit comprises a pressure drop measuring device which is configured to measure the pressure drop between the first pressure measurement port and the second pressure measurement port. The pressure drop measuring device is further configured to carry on signal communications with the transfer unit.

The pressure drop control system for the feed distributor of the fluidized bed reactor provided by the present disclosure is able to measure the pressure drop of the feed distributor accurately. In the system, the transfer unit transfers the signal about a measured pressure drop to the processing unit and the processing unit monitors whether the feed distributor is working normally or not according to the measured pressure drop. The working state of the feed distributor is thus determined precisely. Further, when the processing unit determined that the feed distributor is not working normally, the processing unit performs corresponding processing actions so that the pressure drop of the feed distributor can be restored to the predetermined scope. Specifically, the pressure drop control system of the present disclosure further comprises a nitrogen purging device. When the pressure drop of the feed distributor is larger than the upper limit value of the pressure drop value scope within which the feed distributor is working normally, the processing unit turns on the nitrogen purging device which introduces high-pressure nitrogen gas into the feed distributor so that the feed distributor is purged. By way of this, none of the orifices of the feed distributor is blocked, and the pressure drop of the feed distributor can thus reach a normal value. When the pressure drop of the feed distributor is smaller than the lower limit value of the pressure drop value scope, the nitrogen purging device introduces a high-pressure nitrogen gas into the feed distributor so as to enable the pressure drop of the feed distributor to reach a normal value.

The present disclosure further provides a pressure drop control method for a feed distributor of a fluidized bed reactor. The method comprises the following steps.

In step S1), a pressure drop between a first pressure measurement port and a second pressure measurement port of the feed distributor is measured. The first pressure measurement port is located in an inlet tube of the feed distributor near a reactor wall of the fluidized bed reactor, and the second pressure measurement port is located in an area of the reactor wall between an air distribution plate of the fluidized bed reactor and a gas outlet of a nozzle of the feed distributor.

In step S2), a pressure drop of the feed distributor is calculated according to the pressure drop between the first pressure measurement port and the second pressure measurement port, and it is determined whether the feed distributor is working normally or not. When the pressure drop of the feed distributor is in a predetermined scope within which the pressure drop of the feed distributor is 25%-160% of a pressure drop of a bed of the fluidized bed reactor, the processing unit determines that the feed distributor is working normally. When the processing unit determines that the pressure drop of the feed distributor is larger than an upper limit value or is smaller than a lower limit value of the predetermined scope, the processing unit performs corresponding actions so as to restore the pressure drop of the feed distributor to a value within the predetermined scope. Furthermore, when it is determined that the pressure drop of the feed distributor is smaller than the lower limit value of the predetermined scope, a nitrogen gas is fed into the feed distributor until the pressure drop of the feed distributor is restored to a value within the predetermined scope.

According to the pressure drop control method, when the pressure drop of the feed distributor is in a predetermined scope within which the pressure drop of the feed distributor is 35%-140% of the pressure drop of the bed of the fluidized bed reactor, it is determined that the feed distributor is working normally.

According to the pressure drop control method, in step S2), when it is determined that the pressure drop of the feed distributor is larger than the upper limit value of the predetermined scope, a nitrogen gas is fed into the feed distributor so that the feed distributor can be purged.

According to the pressure drop control method, in step S1), a purging gas having a volumetric flow rate of 0-10 Nm³/h is supplied at the first pressure measurement port and the second pressure measurement port.

According to the pressure drop control method, in step S1), a purging gas having a volumetric flow rate of 1-10 Nm³/h is supplied at the first pressure measurement port and the second pressure measurement port.

The pressure drop control method provided by the present disclosure is capable of measuring the pressure drop of the feed distributor, and determining whether the feed distributor is working normally or not according to the measured pressure drop. By way of this, the working state of the feed distributor is decided accurately. When it is determined that the pressure drop of the feed distributor is larger than the upper limit value or is smaller than the lower limit value of the predetermined scope, corresponding processing actions are performed so that the pressure drop of the feed distributor can be restored to a value within the predetermined scope.

### Brief Description of the Drawings

The accompanying drawings are provided for explaining the present disclosure, not for limiting the scope of the present application in any manner. Shapes and sizes of components in the drawings are merely exemplary and are intended for understanding of the present disclosure, rather than limiting the specific shapes and sizes of the components. Those skilled in the art can implement the present disclosure by selecting possible shapes and sizes of the components as required with teachings of the present disclosure.
Fig. 1 schematically shows a propylene ammoxidation fluidized bed reactor in the prior art;
Fig. 2 schematically shows a nozzle of a propylene and ammonia feed distributor in the prior art;
Fig. 3 schematically shows a pressure drop monitoring system of a feed distributor in the present disclosure; and
Fig. 4 schematically shows a pressure drop monitoring system of a feed distributor in the present disclosure.

In the drawings, same components are designated with same reference numerals. The drawings are not drawn to actual scale.

List of reference numerals:
For the prior art:
   - 1: propylene ammoxidation fluidized bed reactor
   - 10: propylene and ammonia feed distributor
   - 40: reactor wall
   - 60: air distribution plate
   - 70: cooling coil
   - 80: air feed port
   - 12: branch tube
   - 13: orifice
   - 14: nozzle
For the present disclosure:
   - 100: propylene ammoxidation fluidized bed reactor
   - 2: first pressure measurement port
   - 3: second pressure measurement port
   - 4: reactor wall
   - 5: measuring unit
   - 6: air distribution plate
   - 7: cooling coil
   - 8: air feed port
   - 9: high-pressure nitrogen purging device
   - 10: feed distributor

### Detailed Description of the Embodiments

The present disclosure will be explained in detail in conjunction with the accompanying drawings.

It is required generally that a fluidized bed reactor have equal amounts of gas flow in its unit sectional areas. In other words, it is required that all feed gases flowing out of nozzles of a feed distributor are the same in amount, i.e. a mixture gas of propylene and ammonia has a same flow rate when it flows through various orifices. However, it is difficult to measure and monitor the flow rate of the mixture gas when it passes through the orifices. It is therefore difficult to obtain the working state of the reactor directly from the flow rate of the gas when it flows through the orifices.

The inventor of the present disclosure has found from long-term researches that a pressure drop ΔP_{d} of the feed distributor is related to the flow rate of the mixture gas when the mixture gas flows through the orifices. During normal operation of the reactor, in the case that some of the orifices of the feed distributor are blocked, amounts of gases flowing through other orifices are increased, which enables an average rate of the gases (also called average flow rate of the gas passing through the orifice) flowing through these orifices to be increased. This result is represented by an increase of the pressure drop ΔP_{d} of the feed distribution plate. When tubes of the feed distributor suffer from nitrogen embrittlement, more feed gas passes through cracks in the tubes and enter into a bed of the reactor, which reduces the amount of the gas flowing through the orifices and further reduces the average flow rate of the gas passing through the orifices. This result is presented by a decrease of the pressure drop ΔP_{d} of the feed distribution plate.

As aforementioned, in Fig. 2, the pressure difference between the branch 12 and the gas outlet (also the end) of the nozzle 15 is the pressure drop ΔP_{d} of the propylene and ammonia feed distributor. The pressure drop ΔP_{d} of the propylene and ammonia feed distributor is also a pressure difference between a gas inlet of the orifice 13 of the branch 12 and the end of the nozzle 15. However, during a practical production process, it is difficult to measure the pressure difference between the components as shown in Fig. 2. This is because: first, a reaction temperature of the reactor is high and it is usually above 400°C, which can cause damage to a sensor, and second, the arrangement of a sensor in the feed distributor can affect feeding effects of the feed distributor and can easily lead to feed distribution nonuniformity.

It is found through researches and experiments that pressure losses of the mixture gas of propylene and ammonia are very small when the mixture gas flows from an inlet of the feed distributor 10 to the branch 12 (the specific position shown in Fig. 12) and when it enters into the catalyst bed through the nozzle 15. These small pressure losses can be neglected compared with the pressure drop ΔP_{d} of the feed distributor as defined above. It is based on this finding that a pressure drop ΔP_{d} monitoring device and method for a feed distributor of the present disclosure is provided.

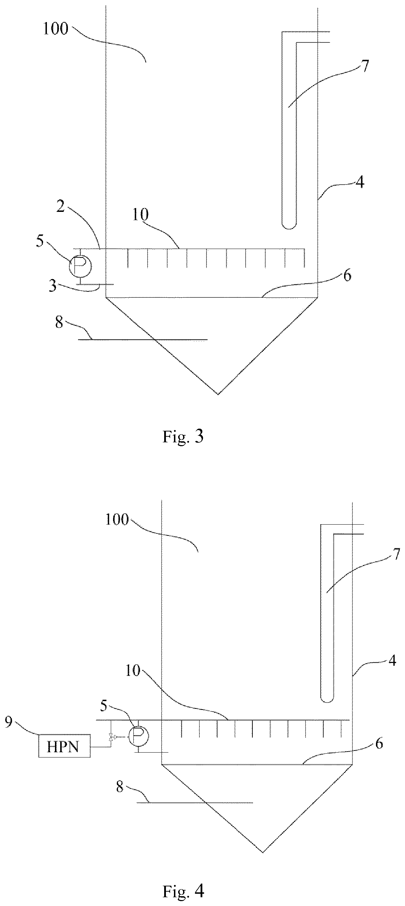

Specifically, as shown in Fig. 4, a pressure drop control system of a feed distributor of a fluidized bed reactor provided by the present disclosure comprises a pressure drop measuring unit (also called pressure drop detector), a transfer unit, and a processing unit. When the pressure drop processing unit detects that a pressure drop of the feed distributor is not in a predetermined scope, the processing unit performs a corresponding processing action so that the pressure drop of the feed distributor is restored to the predetermined scope. By way of this, the pressure drop of the feed distributor is controlled and the distributor is thus ensured to work normally.

Specifically, as shown in Figs. 3 and 4, the pressure drop ΔP_{d} control system of the feed distributor 10 of the present disclosure comprises a measuring unit 5, a transfer unit and a processing unit (not shown in the figures). The measuring unit 5 is configured to measure a pressure drop between a first pressure measurement port 2 and a second pressure measurement port 3. Said pressure drop is taken as the pressure drop ΔP_{d} of the feed distributor 10. The transfer unit is configured to receive the pressure drop measured by the measuring unit 5, and the processing unit is configured to monitor if the feed distributor is working normally or not according to the pressure drop (the pressure drop ΔP_{d} of the feed distributor 10) measured by the measuring unit 5. If the feed distributor is not working normally, the processing unit performs a corresponding processing action so as to enable the feed distributor to work normally.

Preferably, the first pressure measurement port 2 is provided in an inlet tube of the feed distributor 10 near a reactor wall 4, for measurement of a pressure of a mixture gas of propylene and ammonia at an inlet of the feed distributor 10. The second pressure measurement port 3 is preferably located at a part of the reactor wall between an air distribution plate 6 and an end of a nozzle 15 of the feed distributor 10, for measurement of a pressure of the mixture gas of propylene and ammonia at an outlet of the nozzle 15 of the feed distributor 10. Types of the measuring unit 5 are not specifically defined. Different pressure meters can be used to measure the pressures of the mixture gas at the first pressure measurement port 2 and the second pressure measurement port 3, and pressure data signals of the pressure meters are then sent to the transfer unit. The processing unit then calculates a difference between the two pressures by means of logical differential pressure calculation, thus obtaining the pressure drop ΔP_{d} of the feed distributor 10. By providing the pressure meters respectively in the inlet tube of the feed distributor 10 near the reactor wall 4 and in the part of the reactor wall between the air distribution plate 6 and the end of the nozzle 15 of the feed distributor 10, the pressures of the mixture gas at the inlet of the distributor 10 and at the outlet of the nozzle 15 of the feed distributor 10 can be measured accurately. A difference between the two pressures is then taken as the pressure drop ΔP_{d} of the feed distributor 10. The pressure drop ΔP_{d} of the feed distributor 10 is thus measured precisely. In addition, because temperatures at the first pressure measurement port 2 and the second pressure measurement port 3 are relatively low, the pressure meters will not be damaged and the feeding effect of the feed distributor will not be affected. Alternatively, a differential pressure gauge can be used to directly measure the pressure drop between the first pressure measurement port 2 and the second pressure measurement port 3 and transfer a pressure drop data signal to the transfer unit.

Types of the processing unit are not specifically defined. Preferably, the processing unit is a distributed control system (DCS control system) which can show visually perceptible pressure drop variations, i.e. variations of the pressure drop ΔP_{d} of the feed distributor 10.

Preferably, in order to avoid blockage of the pressure measurement ports by a catalyst, the first pressure measurement port 2 and the second pressure measurement port 3 are respectively provided with a same purging gas under same operating conditions (not shown in the figures), the purging gas having a volumetric flow rate of 0-10 Nm³/h, preferably 1-10 Nm³/h, by means of which the pressure measurement ports can be prevented from being blocked by the catalyst. Because the first pressure measurement port 2 and the second pressure measurement port 3 are provided with same purging gases at same operating conditions, the purging gases do not affect results of the pressure drop measurement of the feed distributor. In the case that a speed of the purging gas is too large, it may have an effect on a catalyst bed.

Further, as shown in Fig. 4, the pressure drop control system of the present disclosure further comprises a high-pressure nitrogen purging device 9 (HPN device) which is connected to the feed distributor 10. When the DCS control system determines that a measured value of the pressure drop ΔP_{d} of the feed distributor is not in the predetermined scope, the high-pressure nitrogen purging device is automatically turned on and a high-pressure nitrogen gas is introduced into the feed distributor 10.

The pressure drop ΔP_{d} control system of the feed distributor of the present disclosure is capable of measuring the pressure drop ΔP_{d} of the feed distributor and monitoring if the feed distributor is working normally or not. In a process of producing acrylonitrile, an aperture of an orifice of the feed distributor affects a flow rate variation of a feed gas passing through the orifice, and this kind of effect manifests itself as the variation of the pressure drop ΔP_{d} of the feed distributor. It is therefore feasible to determine whether the feed distributor is working normally or not by monitoring the variation of the pressure drop ΔP_{d} of the feed distribution plate.

During normal operation of the fluidized bed reactor 100, when some orifices of the feed distributor are blocked, amounts of gas passing through other orifices are increased and therefore an average flow rate of the gas passing through these orifices is increased. This result manifests itself as the increase of the pressure drop ΔP_{d} of the feed distributor 10. When tubes of the feed distributor suffer from nitrogen embrittlement, more feed gas passes through cracks in the tubes and enter into the bed, which reduces the amount of the gas flowing through the orifices and further reduces the average flow rate of the gas passing through the orifices. This result is presented by a decrease of the pressure drop ΔP_{d} of the feed distribution plate. The increase or decrease of the pressure drop of the feed distributor indicates that the feed distributor is not working normally.

Specifically, in the system of monitoring the pressure drop ΔP_{d} of the feed distributor of the present disclosure, the pressure data obtained by the measuring unit is transferred to the DCS control system. When the measured value of the pressure drop ΔP_{d} of the feed distributor is 25%∼160% of a pressure drop ΔP_{b} of the bed, it is determined that the feed distributor is working normally. The measured value of the pressure drop ΔP_{d} of the feed distributor is preferably 30%∼150%, or more preferably 35%∼140% of the pressure drop ΔP_{b} of the bed.

On the contrary, if the measured value of the pressure drop ΔP_{d} of the feed distributor is not in the above scope, it is determined that the feed distributor 10 is not working normally.

Specifically, during operation of the fluidized bed reactor 100, when the measured value of the pressure drop ΔP_{d} of the feed distributor is larger than an upper limit value in the above scope, the DCS control system issues an upper limit warning indicating possible blockage of some of the orifices of the feed distributor 10 and automatically turns on the high-pressure nitrogen purging device 9 to purge the feed distributor by introducing the high-pressure nitrogen gas into the feed distributor 10. The blockage is thus cleared. When the measured value of the pressure drop ΔP_{d} of the feed distributor is smaller than a lower limit value in the above scope, the DCS control system issues a lower limit warning indicating possible nitrogen embrittlement of the propylene and ammonia feed distributor.

During over-load operation of the fluidized bed reactor 100, amounts of the feed gas flowing out of all the orifices of the feed distributor are increased at the same compared with full-load operation of the fluidized bed reactor 100. For a same device, the flow rates of the gas when it passes through the orifices are also increased, and the pressure drop ΔP_{d} of the feed distributor is also increased. In the system of monitoring the pressure drop ΔP_{d} of the feed distributor of the present disclosure, the pressure data obtained by the measuring unit is transferred to the DCS control system. The DCS control system issues an upper limit warning. The pressure drop ΔP_{d} of the feed distributor should not be set to be larger than the predetermined upper limit value. For example, the upper limit value can be set to be 160% of the pressure drop ΔP_{b} of the bed of the reactor.

During low-load operation of the fluidized bed reactor 100, amounts of the feed gas flowing out of all the orifices of the feed distributor are decreased at the same time compared with full-load operation of the fluidized bed reactor 100. For a same fluidized bed reactor 100, the flow rates of the gas when it passes through the orifices are also decreased, which is presented by the decrease of the pressure drop ΔP_{d} of the feed distributor. In an extremely severe case, gas distribution effects of the feed distributor are affected and non-uniformity of gas feeding is resulted in. Results of the reaction are thus deteriorated. In the system of monitoring the pressure drop ΔP_{d} of the feed distributor of the present disclosure, the pressure data obtained by the measuring unit is transferred to the DCS control system. The DCS control system issues a lower limit warning. For example, the lower limit value can be set to be 20% of the pressure drop ΔP_{b} of the bed of the fluidized bed reactor. During low-load operation of the fluidized bed reactor 100, when the pressure drop ΔP_{d} of the feed distributor is smaller than the lower limit value, the high-pressure nitrogen purging device is automatically turned on and the nitrogen gas flow can be adjusted, so that the nitrogen gas can be introduced into feed gas tubes steadily and continuously and be fed into the feed distributor 10 together with the feed gas. In this way, the pressure drop ΔP_{d} of the feed distributor can be enabled to reach the predetermined upper lower limit set by the DCS control system, and the reactor can thus work normally.

### Embodiments

The pressure drop ΔP_{d} monitoring system of the feed distributor provided by the present disclosure will be described in a more detailed way below. The present disclosure is not limited to the following embodiments.

### Embodiment 1

A fluidized bed reactor for producing acrylonitrile having a diameter of 7 m was used. A catalyst used was commonly used commercial acrylonitrile catalyst. A molar ratio of propylene/ammonia/air in a feed gas was 1:1.1:9.3. A reaction temperature was 440°C, and a reaction pressure was 0.5 kg/m³. The reactor was operated at full load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 41.6% of a pressure drop ΔP_{b} of a bed of the fluidized bed reactor. Yield of AN was 80.2%, and conversion rate of propylene was 98.0%.

### Embodiment 2

A fluidized bed reactor for producing acrylonitrile and reaction conditions were the same as those used in Embodiment 1. The reactor was operated at 80% load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 26.8% of a pressure drop ΔP_{b} of a bed of the fluidized bed reactor. Yield of AN was 79.7%, and conversion rate of propylene was 97.5%.

### Embodiment 3

A fluidized bed reactor for producing acrylonitrile and reaction conditions were the same as those used in Embodiment 1. The reactor was operated at 70% load. Pressure measurement ports were provided in an inlet tube of a feed distributor near a reactor wall and in a part of the reactor wall between an air distribution plate and an end of a nozzle of the feed distributor, respectively. A high-pressure nitrogen purging device was turned on, and nitrogen gas was introduced into the feed distributor. A pressure drop ΔP_{d} of the propylene and ammonia feed distributor measured practically was 41.4% of a pressure drop ΔP_{b} of a bed of the fluidized bed reactor. Yield of AN was 80.0%, and conversion rate of propylene was 98.6%.

### Comparative Embodiment 1

A fluidized bed reactor for producing acrylonitrile and reaction conditions were the same as those used in Embodiment 1. The reactor was operated at full load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 21.8% of a pressure drop ΔP_{b} of a bed of the fluidized bed reactor. Yield of AN was 79.4%, and conversion rate of propylene was 97.1%.

### Comparative Embodiment 2

A fluidized bed reactor for producing acrylonitrile and reaction conditions were the same as those used in Embodiment 1. The reactor was operated at full load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 18.2% of a pressure drop ΔP_{b} of a bed of the fluidized bed reactor. Yield of AN was 75.3%, and conversion rate of propylene was 93.2%. The reactor was stopped for overhaul. It was found that there were leaks in tubes of the feed distributor caused by embrittlement.

### Embodiment 4

A fluidized bed reactor for producing acrylonitrile having a diameter of 12 m was used. A catalyst used was the same as the acrylonitrile catalyst used in Embodiment 1. A molar ratio of propylene/ammonia/air in a feed gas was 1:1.1:9.3. A reaction temperature was 440°C, and a reaction pressure was 0.5 kg/m³. The reactor was operated at full load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 42.6% of a pressure drop ΔP_{b} of a bed of the fluidized bed reactor. Yield of AN was 80.1%, and conversion rate of propylene was 98.4%.

### Embodiment 5

A fluidized bed reactor for producing acrylonitrile and reaction conditions were the same as those used in Embodiment 4. The reactor was operated at 70% load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 25.8% of a pressure drop ΔP_{b} of a bed of the fluidized bed reactor. Yield of AN was 79.3%, and conversion rate of propylene was 96.5%.

### Embodiment 6

A fluidized bed reactor for producing acrylonitrile and reaction conditions were the same as those used in Embodiment 4. The reactor was operated at 70% load. Pressure measurement ports were provided in an inlet tube of a feed distributor near a reactor wall and in a part of the reactor wall between an air distribution plate and an end of a nozzle of the feed distributor, respectively. A high-pressure nitrogen purging device was turned on, and nitrogen gas was introduced into the feed distributor. A pressure drop ΔP_{d} of the propylene and ammonia feed distributor measured practically was 38.8% of a pressure drop ΔP_{b} of a bed of the fluidized bed reactor. Yield of AN was 79.8%, and conversion rate of propylene was 98.2%.

### Embodiment 7

A fluidized bed reactor for producing acrylonitrile having a diameter of 9 m was used. A catalyst used was the same as the acrylonitrile catalyst used in Embodiment 1. A molar ratio of propylene/ammonia/air in a feed gas was 1:1.1:9.3. A reaction temperature was 440°C, and a reaction pressure was 0.5 kg/m³. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 90.9% of a pressure drop ΔP_{b} of a bed of the fluidized bed reactor. Yield of AN was 80.5%, and conversion rate of propylene was 99.1%.

### Embodiment 8

A fluidized bed reactor for acrylonitrile having a diameter of 12 m was used. A catalyst used was the same as the acrylonitrile catalyst used in Embodiment 1. A molar ratio of propylene/ammonia/air in a feed gas was 1:1.1:9.3. A reaction temperature was 440°C, and a reaction pressure was 0.5 kg/m³. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 98.8% of a pressure drop ΔP_{b} of a bed of the fluidized bed reactor. Yield of AN was 80.3%, and conversion rate of propylene was 98.7%.

### Embodiment 9

A fluidized bed reactor for producing acrylonitrile and reaction conditions were the same as those used in Embodiment 8. The reactor was operated at full load. Pressure measurement ports were provided in an inlet tube of a feed distributor near a reactor wall and in a part of the reactor wall between an air distribution plate and an end of a nozzle of the feed distributor, respectively. A pressure drop ΔP_{d} of the propylene and ammonia feed distributor measured practically was 172.4% of a pressure drop ΔP_{b} of a bed of the fluidized bed reactor. Yield of AN was 78.7%, and conversion rate of propylene was 96.5%. A DCS control system automatically turned on a high-pressure nitrogen purging device, and nitrogen gas was introduced into the feed distributor for purging. After the nitrogen purging device was turned off and the reaction became stable, it was obtained through measurement that the pressure drop ΔP_{d} of the propylene and ammonia feed distributor was 103.1% of the pressure drop ΔP_{b} of the bed of the fluidized bed reactor. Yield of AN was 80.4%, and conversion rate of propylene was 98.7%.

The above details are only descriptions of preferred embodiments of the present disclosure.

## Claims

1. A pressure drop control system for a feed distributor of a fluidized bed ammoxidation reactor, the pressure drop control system comprises:
a measuring unit which is configured to measure a pressure drop between a first pressure measurement port and a second pressure measurement port of the feed distributor,
wherein the first pressure measurement port is located in an inlet tube of the feed distributor near a reactor wall of the fluidized bed reactor, and the second pressure measurement port is located in an area of the reactor wall between an air distribution plate of the fluidized bed reactor and a gas outlet of a nozzle of the feed distributor,
**characterized in that**
a transfer unit which is configured to carry on signal communications with the measuring unit and collect a signal about the pressure drop measured by the measuring unit, and
a processing unit which is configured to monitor whether the feed distributor is working normally or not,
wherein when a pressure drop of the feed distributor is in a predetermined scope within which the pressure drop of the feed distributor is 25%-160% of a pressure drop of a bed of the fluidized bed reactor, the processing unit determines that the feed distributor is working normally, and
wherein when the processing unit determines that the pressure drop of the feed distributor is larger than an upper limit value or smaller than a lower limit value of the predetermined scope, the processing unit performs corresponding actions so as to restore the pressure drop of the feed distributor to a value within the predetermined scope, and
the pressure drop control system further comprises a nitrogen purging device, when the processing unit determines that the pressure drop of the feed distributor is larger than the upper limit value of the predetermined scope, the processing unit is configured to turn on the nitrogen purging device so that the feed distributor can be purged.

2. The pressure drop control system according to claim 1, further comprising purging units which are provided at the first pressure measurement port and the second pressure measurement port respectively.

3. The pressure drop control system according to claim 2, wherein each of the purging units is configured to supply a purging gas having a volumetric flow rate smaller than or equal to 10 Nm³/h.

4. The pressure drop control system according to claim 1, wherein when the pressure drop of the feed distributor is in a predetermined scope within which the pressure drop of the feed distributor is 35%-140% of the pressure drop of the bed of the fluidized bed reactor, the processing unit determines that the feed distributor is working normally.

5. The pressure drop control system according to claim 1, further comprising a nitrogen purging device,
wherein when the processing unit determines that the pressure drop of the feed distributor is smaller than the lower limit value of the predetermined scope, the processing unit turns on the nitrogen purging device so that a nitrogen gas is fed into the feed distributor, thereby restoring the pressure drop of the feed distributor to a value within the predetermined scope.

6. The pressure drop control system according to any of claims 1 to 5,
wherein the measuring unit comprises a first pressure measurement device that is provided at the first pressure measurement port and a second pressure measurement device that is provided at the second pressure measurement port, and
wherein the transfer unit is configured to carry on signal communications with the first pressure measurement device and the second pressure measurement device respectively, and the processing unit is configured to perform logical differential pressure calculation so as to obtain the pressure drop of the feed distributor.

7. The pressure drop control system according to any of claims 1 to 5, wherein the measuring unit comprises a pressure drop measuring device which is configured to measure the pressure drop between the first pressure measurement port and the second pressure measurement port, wherein the pressure drop measuring device is further configured to carry on signal communications with the transfer unit.

8. A pressure drop control method for a feed distributor of a fluidized bed ammoxidation reactor, comprising steps of:
S1): measuring a pressure drop between a first pressure measurement port and a second pressure measurement port of the feed distributor, wherein the first pressure measurement port is located in an inlet tube of the feed distributor near a reactor wall of the fluidized bed reactor, and the second pressure measurement port is located in an area of the reactor wall between an air distribution plate of the fluidized bed reactor and a gas outlet of a nozzle of the feed distributor,
wherein the method is **characterized by**
S2): calculating a pressure drop of the feed distributor according to the pressure drop between the first pressure measurement port and the second pressure measurement port, and determining whether the feed distributor is working normally or not,
wherein when the pressure drop of the feed distributor is in a predetermined scope within which the pressure drop of the feed distributor is 25%-160% of a pressure drop of a bed of the fluidized bed reactor, it is determined that the feed distributor is working normally, and
wherein when it is determined that the pressure drop of the feed distributor is larger than an upper limit value or smaller than a lower limit value of the predetermined scope, corresponding actions are performed so as to restore the pressure drop of the feed distributor to a value within the predetermined scope, and
wherein when it is determined that the pressure drop of the feed distributor is larger than the upper limit value of the predetermined scope, a nitrogen gas is fed into the feed distributor so that the feed distributor can be purged.

9. The pressure drop control method according to claim 8, wherein when the pressure drop of the feed distributor is in a predetermined scope within which the pressure drop of the feed distributor is 35%-140% of the pressure drop of the bed of the fluidized bed reactor, it is determined that the feed distributor is working normally.

10. The pressure drop control method according to claim 8 or 9, wherein in S2), when it is determined that the pressure drop of the feed distributor is smaller than the lower limit value of the predetermined scope, a nitrogen gas is fed into the feed distributor until the pressure drop of the feed distributor is restored to a value within the predetermined scope.

11. The pressure drop control method according to claim 8, wherein in S1), a purging gas having a volumetric flow rate of 0-10 Nm³/h is supplied at the first pressure measurement port and the second pressure measurement port.

12. The pressure drop control method according to claim 11, wherein in S1), a purging gas having a volumetric flow rate of 1-10 Nm³/h is supplied at the first pressure measurement port and the second pressure measurement port.

## Patentansprüche

1. Ein Druckabfall-Steuersystem für einen Beschickungsverteiler eines Wirbelschicht-Ammoxidationsreaktors, wobei das Druckabfall-Steuersystem umfasst:
eine Messeinheit, die so konfiguriert ist, dass sie einen Druckabfall zwischen einem ersten Druckmessanschluss und einem zweiten Druckmessanschluss des Beschickungsverteilers misst,
wobei der erste Druckmessanschluss in einem Einlassrohr des Beschickungsverteilers nahe der Reaktorwand des Wirbelschichtreaktors angeordnet ist, und der zweite Druckmessanschluss in einem Bereich der Reaktorwand zwischen einer Luftverteilungsplatte des Wirbelschichtreaktors und einem Gasauslass einer Düse des Beschickungsverteilers angeordnet ist,
**gekennzeichnet durch**
eine Übertragungseinheit, die so konfiguriert ist, dass sie Signalkommunikation mit der Messeinheit betreibt und ein Signal über den von der Messeinheit gemessenen Druckabfall erfasst, und
eine Verarbeitungseinheit, die so konfiguriert ist, dass sie überwacht, ob der Beschickungsverteiler normal arbeitet oder nicht,
wobei, wenn der Druckabfall des Beschickungsverteilers in einem vorbestimmten Bereich liegt, innerhalb dessen der Druckabfall des Beschickungsverteilers 25%-160% eines Druckabfalls eines Bettes des Wirbelschichtreaktors beträgt, die Verarbeitungseinheit feststellt, dass der Beschickungsverteiler normal arbeitet, und
wobei, wenn die Verarbeitungseinheit feststellt, dass der Druckabfall des Beschickungsverteilers größer als ein oberer Grenzwert oder kleiner als ein unterer Grenzwert des vorbestimmten Bereichs ist, die Verarbeitungseinheit entsprechende Maßnahmen durchführt, um den Druckabfall des Beschickungsverteilers auf einen Wert innerhalb des vorbestimmten Bereichs zurückzuführen, und
das Druckabfall-Steuersystem ferner eine Stickstoffspülvorrichtung umfasst, wobei die Verarbeitungseinheit, wenn sie feststellt, dass der Druckabfall des Beschickungsverteilers größer ist als der obere Grenzwert des vorgegebenen Bereichs, so konfiguriert ist, dass sie die Stickstoffspülvorrichtung einschaltet, so dass der Zufuhrverteiler gespült werden kann.

2. Das Druckabfall-Steuersystem nach Anspruch 1, das ferner Spüleinheiten umfasst, die am ersten Druckmessanschluss bzw. am zweiten Druckmessanschluss vorgesehen sind.

3. Das Druckabfall-Steuersystem nach Anspruch 2, wobei jede der Spüleinheiten so konfiguriert ist, dass sie ein Spülgas mit einem Volumenstrom kleiner oder gleich 10 Nm³/h liefert.

4. Das Druckabfall-Steuersystem nach Anspruch 1, wobei, wenn der Druckabfall des Beschickungsverteilers in einem vorbestimmten Bereich liegt, innerhalb dessen der Druckabfall des Zufuhrverteilers 35%-140% des Druckabfalls des Bettes des Wirbelschichtreaktors beträgt, die Verarbeitungseinheit feststellt, dass der Zufuhrverteiler normal arbeitet.

5. Das Druckabfall-Steuersystem nach Anspruch 1, das ferner eine Stickstoffspülvorrichtung umfasst,
wobei, wenn die Verarbeitungseinheit feststellt, dass der Druckabfall des Beschickungsverteilers kleiner als der untere Grenzwert des vorbestimmten Bereichs ist, die Verarbeitungseinheit die Stickstoffspülvorrichtung einschaltet, so dass Stickstoffgas in den Beschickungsverteiler eingespeist wird, wodurch der Druckabfall des Beschickungsverteilers wieder auf einen Wert innerhalb des vorbestimmten Bereichs gebracht wird.

6. Das Druckabfall-Steuersystem nach einem der Ansprüche 1 bis 5,
wobei die Messeinheit eine erste Druckmessvorrichtung, die an dem ersten Druckmessanschluss vorgesehen ist, und eine zweite Druckmessvorrichtung, die an dem zweiten Druckmessanschluss vorgesehen ist, umfasst, und
wobei die Übertragungseinheit so konfiguriert ist, dass sie Signalkommunikation mit der ersten Druckmessvorrichtung bzw. der zweiten Druckmessvorrichtung betreibt, und die Verarbeitungseinheit so konfiguriert ist, dass sie eine logische Differenzdruckberechnung durchführt, um den Druckabfall des Beschickungsverteilers zu erhalten.

7. Das Druckabfall-Steuersystem nach einem der Ansprüche 1 bis 5, wobei die Messeinheit eine Druckabfallmessvorrichtung umfasst, die so konfiguriert ist, dass sie den Druckabfall zwischen dem ersten Druckmessanschluss und dem zweiten Druckmessanschluss misst, wobei die Druckabfallmessvorrichtung ferner so konfiguriert ist, dass sie Signalkommunikation mit der Übertragungseinheit betreibt.

8. Ein Verfahren zur Regelung des Druckabfalls für einen Beschickungsverteiler eines Wirbelschicht-Ammoxidationsreaktors, welches die folgenden Schritte umfasst:
S1): Messen des Druckabfalls zwischen einem ersten Druckmessanschluss und einem zweiten Druckmessanschluss des Beschickungsverteilers, wobei der erste Druckmessanschluss in einem Einlassrohr des Beschickungsverteilers nahe der Reaktorwand des Wirbelschichtreaktors angeordnet ist, und der zweite Druckmessanschluss in einem Bereich der Reaktorwand zwischen einer Luftverteilungsplatte des Wirbelschichtreaktors und einem Gasauslass einer Düse des Beschickungsverteilers angeordnet ist,
wobei das Verfahren **gekennzeichnet ist durch**
S2): Berechnen des Druckabfalls des Beschickungsverteilers entsprechend dem Druckabfall zwischen dem ersten Druckmessanschluss und dem zweiten Druckmessanschluss, und bestimmen, ob der Beschickungsverteiler normal arbeitet oder nicht,
wobei, wenn der Druckabfall des Beschickungsverteilers in einem vorbestimmten Bereich liegt, innerhalb dessen der Druckabfall des Beschickungsverteilers 25%-160% des Druckabfalls des Bettes des Wirbelschichtreaktors beträgt, festgestellt wird, dass der Beschickungsverteiler normal arbeitet, und
wobei, wenn festgestellt wird, dass der Druckabfall des Beschickungsverteilers größer als ein oberer Grenzwert oder kleiner als ein unterer Grenzwert des vorbestimmten Bereichs ist, entsprechende Maßnahmen durchgeführt werden, um den Druckabfall des Beschickungsverteilers auf einen Wert innerhalb des vorbestimmten Bereichs zurückzuführen, und
wobei, wenn festgestellt wird, dass der Druckabfall des Beschickungsverteilers größer ist als der obere Grenzwert des vorgegebenen Bereichs, Stickstoffgas in den Beschickungsverteiler eingespeist wird, so dass der Beschickungsverteiler gespült werden kann.

9. Das Verfahren zur Regelung des Druckabfalls nach Anspruch 8, wobei, wenn der Druckabfall des Beschickungsverteilers in einem vorbestimmten Bereich liegt, in dem der Druckabfall des Beschickungsverteilers 35%-140% des Druckabfalls des Bettes des Wirbelschichtreaktors beträgt, festgestellt wird, dass der Beschickungsverteiler normal arbeitet.

10. Das Verfahren zur Regelung des Druckabfalls nach Anspruch 8 oder 9, wobei in S2), wenn festgestellt wird, dass der Druckabfall des Beschickungsverteilers kleiner als der untere Grenzwert des vorbestimmten Bereichs ist, Stickstoffgas in den Beschickungsverteiler eingespeist wird, bis der Druckabfall des Beschickungsverteilers auf einen Wert innerhalb des vorbestimmten Bereichs zurückgeführt ist.

11. Das Verfahren zur Regelung des Druckabfalls nach Anspruch 8, wobei in S1) ein Spülgas mit einem Volumenstrom von 0-10 Nm³/h über den ersten Druckmessanschluss und über den zweiten Druckmessanschluss zugeführt wird.

12. Das Verfahren zur Regelung des Druckabfalls nach Anspruch 11, wobei in S1) ein Spülgas mit einem Volumenstrom von 1-10 Nm³/h über den ersten Druckmessanschluss und über den zweiten Druckmessanschluss zugeführt wird.

## Revendications

1. Système de commande de chute de pression pour un distributeur d'alimentation d'un réacteur d'ammoxydation à lit fluidisé, le système de commande de chute de pression comprend :
une unité de mesure qui est configurée pour mesurer une chute de pression entre un premier orifice de mesure de pression et un second orifice de mesure de pression du distributeur d'alimentation,
dans lequel le premier orifice de mesure de pression est situé dans un tube d'entrée du distributeur d'alimentation près d'une paroi de réacteur du réacteur à lit fluidisé, et le second orifice de mesure de pression est situé dans une zone de la paroi de réacteur entre une plaque de distribution d'air du réacteur à lit fluidisé et une sortie de gaz d'une buse du distributeur d'alimentation,
**caractérisé en ce que**
une unité de transfert qui est configurée pour réaliser des communications de signal avec l'unité de mesure et collecter un signal concernant la chute de pression mesurée par l'unité de mesure, et
une unité de traitement qui est configurée pour surveiller le fait que le distributeur d'alimentation fonctionne normalement ou non,
dans lequel, lorsqu'une chute de pression du distributeur d'alimentation est dans une plage prédéterminée au sein de laquelle la chute de pression du distributeur d'alimentation est de 25 % à 160 % d'une chute de pression d'un lit du réacteur à lit fluidisé, l'unité de traitement détermine que le distributeur d'alimentation fonctionne normalement, et
dans lequel, lorsque l'unité de traitement détermine que la chute de pression du distributeur d'alimentation est plus grande qu'une valeur de limite supérieure ou plus petite qu'une valeur de limite inférieure de la plage prédéterminée, l'unité de traitement effectue des actions correspondantes afin de remettre la chute de pression du distributeur d'alimentation à une valeur au sein de la plage prédéterminée, et
le système de commande de chute de pression comprend en outre un dispositif de purge d'azote, lorsque l'unité de traitement détermine que la chute de pression du distributeur d'alimentation est plus grande que la valeur de limite supérieure de la plage prédéterminée, l'unité de traitement est configurée pour allumer le dispositif de purge d'azote pour que le distributeur d'alimentation puisse être purgé.

2. Système de commande de chute de pression selon la revendication 1, comprenant en outre des unités de purge qui sont prévues au premier orifice de mesure de pression et au second orifice de mesure de pression respectivement.

3. Système de commande de chute de pression selon la revendication 2, dans lequel chacune des unités de purge est configurée pour effectuer l'alimentation en un gaz de purge ayant un débit volumétrique inférieur ou égal à 10 Nm³/h.

4. Système de commande de chute de pression selon la revendication 1, dans lequel, lorsque la chute de pression du distributeur d'alimentation est dans une plage prédéterminée au sein de laquelle la chute de pression du distributeur d'alimentation est de 35 % à 140 % de la chute de pression du lit du réacteur à lit fluidisé, l'unité de traitement détermine que le distributeur d'alimentation fonctionne normalement.

5. Système de commande de chute de pression selon la revendication 1, comprenant en outre un dispositif de purge d'azote,
dans lequel, lorsque l'unité de traitement détermine que la chute de pression du distributeur d'alimentation est plus petite que la valeur de limite inférieure de la plage prédéterminée, l'unité de traitement allume le dispositif de purge d'azote pour qu'un azote gazeux soit fourni dans le distributeur d'alimentation, ainsi remettant la chute de pression du distributeur d'alimentation à une valeur au sein de la plage prédéterminée.

6. Système de commande de chute de pression selon l'une quelconque des revendications 1 à 5,
dans lequel l'unité de mesure comprend un premier dispositif de mesure de pression qui est prévu au premier orifice de mesure de pression et un second dispositif de mesure de pression qui est prévu au second orifice de mesure de pression, et
dans lequel l'unité de transfert est configurée pour réaliser des communications de signal avec le premier dispositif de mesure de pression et le second dispositif de mesure de pression respectivement, et l'unité de traitement est configurée pour réaliser un calcul de pression différentielle logique afin d'obtenir la chute de pression du distributeur d'alimentation.

7. Système de commande de chute de pression selon l'une quelconque des revendications 1 à 5, dans lequel l'unité de mesure comprend un dispositif de mesure de chute de pression qui est configuré pour mesurer la chute de pression entre le premier orifice de mesure de pression et le second orifice de mesure de pression, dans lequel la dispositif de mesure de chute de pression est en outre configuré pour réaliser des communications de signal avec l'unité de transfert.

8. Procédé de commande de chute de pression pour un distributeur d'alimentation d'un réacteur d'ammoxydation à lit fluidisé, comprenant des étapes de :
S1) : la mesure d'une chute de pression entre un premier orifice de mesure de pression et un second orifice de mesure de pression du distributeur d'alimentation, dans lequel le premier orifice de mesure de pression est situé dans un tube d'entrée du distributeur d'alimentation près d'une paroi de réacteur du réacteur à lit fluidisé, et le second orifice de mesure de pression est situé dans une zone de la paroi de réacteur entre une plaque de distribution d'air du réacteur à lit fluidisé et une sortie de gaz d'une buse du distributeur d'alimentation,
dans lequel le procédé est **caractérisé par**
S2) : le calcul d'une chute de pression du distributeur d'alimentation selon la chute de pression entre le premier orifice de mesure de pression et le second orifice de mesure de pression, et la détermination du fait que le distributeur d'alimentation fonctionne normalement ou non,
dans lequel, lorsque la chute de pression du distributeur d'alimentation est dans une plage prédéterminée au sein de laquelle la chute de pression du distributeur d'alimentation est de 25 % à 160 % d'une chute de pression d'un lit du réacteur à lit fluidisé, il est déterminé que le distributeur d'alimentation fonctionne normalement, et
dans lequel, lorsqu'il est déterminé que la chute de pression du distributeur d'alimentation est plus grande qu'une valeur de limite supérieure ou plus petite qu'une valeur de limite inférieure de la plage prédéterminée, des actions correspondantes sont réalisées afin de remettre la chute de pression du distributeur d'alimentation à une valeur au sein de la plage prédéterminée, et
dans lequel, lorsqu'il est déterminé que la chute de pression du distributeur d'alimentation est plus grande que la valeur de limite supérieure de la plage prédéterminée, un azote gazeux est fourni dans le distributeur d'alimentation pour que le distributeur d'alimentation puisse être purgé.

9. Procédé de commande de chute de pression selon la revendication 8, dans lequel, lorsque la chute de pression du distributeur d'alimentation est dans une plage prédéterminée au sein de laquelle la chute de pression du distributeur d'alimentation est de 35 % à 140 % de la chute de pression du lit du réacteur à lit fluidisé, il est déterminé que le distributeur d'alimentation fonctionne normalement.

10. Procédé de commande de chute de pression selon la revendication 8 ou 9, dans lequel dans S2), lorsqu'il est déterminé que la chute de pression du distributeur d'alimentation est plus petite que la valeur de limite inférieure de la plage prédéterminée, un azote gazeux est fourni dans le distributeur d'alimentation jusqu'à ce que la chute de pression du distributeur d'alimentation soit remise à une valeur au sein de la plage prédéterminée.

11. Procédé de commande de chute de pression selon la revendication 8, dans lequel, dans S1), une alimentation en un gaz de purge ayant un débit volumétrique de 0 à 10 Nm³/h est effectuée au premier orifice de mesure de pression et au second orifice de mesure de pression.

12. Procédé de commande de chute de pression selon la revendication 11, dans lequel, dans S1), une alimentation en un gaz de purge ayant un débit volumétrique de 1 à 10 Nm³/h est effectuée au premier orifice de mesure de pression et au second orifice de mesure de pression.
